Europäisches Patentamt

**European Patent Office**

Office européen des brevets

(11) Publication number: **0 405 511**

A2

(12) **EUROPEAN PATENT APPLICATION**

(21) Application number: **90112272.1**

(22) Date of filing: **27.06.90**

(51) Int. Cl.⁵: **A01H 1/02, A01H 4/00**

---

The microorganism(s) has (have) been deposited with American Type Culture Collection under numbers 40519 and 40520.

(30) Priority: **28.06.89 US 372724**

(43) Date of publication of application:
**02.01.91 Bulletin 91/01**

(84) Designated Contracting States:
**BE CH DE DK ES FR GB IT LI NL SE**

�splicant: **DOWELANCO**
**4040 Vincennes Circle**
**Indianapolis, Indiana 46268(US)**

(72) Inventor: **Mitchell, Jon C.**
**111 E. Healey Apt. 103**
**Champaign, Illinois 61820(US)**
Inventor: **Petolino, Joseph F.**
**703 W. Ohio**
**Urbana, Illinois 61801(US)**

(74) Representative: **Huber, Bernhard, Dipl.-Chem.**
**et al**
**Patentanwälte H. Weickmann, Dr. K. Fincke**
**F.A. Weickmann, B. Huber Dr. H. Liska, Dr. J.**
**Prechtel Möhlstrasse 22 Postfach 860 820**
**D-8000 München 86(DE)**

---

(54) **Method for the suspension culture of haploid maize male gametophytes and products therefrom.**

㊵ A method for the suspension culture of haploid maize male gametophytes and products produced therefrom.

**EP 0 405 511 A2**

# METHOD FOR THE SUSPENSION CULTURE OF HAPLOID MAIZE MALE GAMETOPHYTES AND PRODUCTS THEREFROM

This invention relates to a method for the establishment of a suspension culture. More specifically, this invention relates to a suspension culture of haploid or double haploid maize male gametophytes which are capable of plant regeneration.

It is of great agricultural and economic interest to provide new genotypes of maize plants which display an improvement in particular characteristics. Through proper breeding techniques, these characteristics can be introduced into new or existing genotypes which can then be marketed-directly or used to produce superior hybrid maize plants.

The development of a hybrid variety by selective breeding conventionally involves three steps: (1) the selection of superior plants from various germplasm pools; (2) the selfing of the superior plants for several generations to produce a series of inbred lines, which although different from each other, breed true and are highly uniform; and (3) the crossing of selected inbred lines with unrelated inbred lines to produce the hybrid progeny ($F_1$). An important consequence of the homozygosity and homogeneity of the inbred lines is that the hybrid between any two inbreds will always be the same. Once the inbreds that give the best hybrid have been identified, the hybrid seed can be reproduced indefinitely as long as the homogeneity of the inbred parents are maintained.

Improvements by selective breeding have been relatively slow, since only a limited number of generations of plants may be propagated each year. Therefore, relatively minor improvements in plants have been obtained only after years of rigorous work.

A culture of cells outside the body of the host is termed *"in vitro"*. Isolated spore culture provides a readily available source of free haploid cells, and thus is a promising tool for basic and applied breeding programs (see Sunderland et al. (1977), Nature 270:236-238).

The culture of spores provides a method of inducing homozygosity in plants. By "spore" is meant to include microspores and megaspores.

Microspores normally develop as follows. Following mitosis and after release from the tetrad, each individual microspore is characterized by a large nucleus which occupies one-third to one-half of the cell volume. A germ pore appears prior to enlargement of the microspore while the cytoplasm is dispersed throughout the cell. As the microspore increases in size, the nucleus remains near the pore and becomes smaller and more densely staining. With the formation of a large central vacuole, the nucleus is pushed to the side opposite the germ pore and organizes a separate cell attached to the microspore wall. Within this cell, the nucleus enlarges and undergoes the first pollen grain mitosis. The daughter nuclei, which are initially similar in size, begin to differentiate while still within the original cytoplasm. At this point, the vegetative nucleus is distinguishable by its larger size and more diffuse staining in contrast to the smaller and more densely staining generative nucleus.

The vegetative nucleus migrates to the opposite side of the pollen grain. The generative nucleus, along with a small amount of cytoplasm, is separated from the vegetative nucleus by the formation of an arch-shaped cell wall attached to the intine. Formation of a distinct cell wall around the vegetative nucleus appears to be delayed during migration. Upon reaching a position adjacent to the pore, a cell is formed which is similar to, but larger than, the generative cell. Starch accumulation follows progressing from the tapetal or pore side until the entire grain is filled. The generative cell then detaches from the intine, becomes rounded, and migrates toward the vegetative cell where the second pollen grain mitosis results in a trinucleate pollen grain. For a general description of male gametophyte development, see Pescitelli and Petolino (1988), Plant Cell Reports 7:441-444.

Megaspores normally develop as follows. Within the ovule, the megaspore mother cell divides mitotically to produce four (4) haploid megaspores. Three of these megaspores disintegrate, the fourth divides mitotically, developing into an embryo sac - the female gametophyte. The female gametophyte consists of seven cells with a total of eight haploid nuclei (the large central cell contains two nuclei - the polar nulcei). One of the smaller cells, containing a single haploid nucleus, is the egg cell. For a general discussion, see Biology , Curtis (ed.) (1983), pp. 591-613.

Microspores are male reproductive cells which, along with pollen gametophytes derived from them, may be diverted from their normal development to form homozygous plants by androgenesis. Megaspores are female reproductive cells which may also be diverted from their normal development to form homozygous plants by gynogenesis.

Spores may be diverted from their normal development sequence to develop as embryos or calli from which plants can be regenerated. The resulting cultures are haploid and contain only a single set of chromosomes from the original plants. The plants derived from these cultures are sterile unless chromosome doubling occurs, either spontaneously or by chemical induction, to create doubled haploids which are fully fertile and completely inbred. For a general discussion of anther culture see Dunwell (1985), In: Cereal Tissue and Cell Culture , Bright and Jones, (eds.), pp. 1-44. For a general discussion of ovary culture, see Yang and Zhou (1982), Theor. Appl. Genet. 63:97-104.

Consequently, the culture of spores represents a method by which, theoretically, large numbers of haploid individuals can be produced directly from gametophytic cells in vitro. (see Keller et al., In: Plant Tissue and Cell Culture , Green, et al (eds.), pp 223-241), and Yang and Zhou (1982), supra.

One type of culture is suspension culture, i.e., the cultured cells are not attached to a surface, but instead are continuously or intermittently freely suspended and, optionally, agitated in the culture media. Suspension culture is of great value because the environment around every cell is the same, and each cell can derive its nutrition from, and excrete its waste products into, the media surrounding the entire surface of the cell. Furthermore, by agitating the culture, this allows for aeration of the tissues and for bathing each cell with medium. Suspension culture also breaks up large cell clusters, embryo-like structures (ELS), and callus chunks, maintaining the culture as single cells, small cell clusters, multicellular masses (MM).

To date, studies reporting the suspension culture of maize in the prior art have involved the culture of maize from immature embryo-derived callus. (See Polikarpochkina et al. (1979), Z. Pflanzenphysiol. 95:57-67.) The immature embryo-derived callus, however, has a diploid chromosome complement, which would not provide the benefits of a haploid-derived plant. Thus, isolated gametophytes of maize have not heretofore demonstrated the ability to be suspension cultured.

Suprisingly, the present invention provides ·a method that is capable of proliferating and multiplying cells and aggregates of cells in a suspension medium.

One could not predict from the prior art at the time this invention was made, how to maintain a culture of proliferating cells.

In a first aspect, the present invention provides a method for the culture of maize male gametophytes comprising the step of: (a) providing at least one haploid maize male gametophyte from at least one haploid donor maize plant which is capable of being successfully in vitro cultured, wherein the donor maize plant is produced by (i) providing anthers from at least one heterozygous donor maize plant, (ii) regenerating, from the anthers obtained from the donor plant, at least two microspore-derived plants capable of being intermated, (iii) intermating the regenerated plants to produce an F₁ population, and (iv) self-pollinating or cross-pollinating individuals of the F₁ population to generate at least one F₂ population; (b) culturing a haploid maize male gametophyte in a suspension medium, said medium containing components which allow for the cells of the male gametophyte to actively grow and divide, and to maintain their development as single cells or cell clusters.

In a second aspect, the present invention provides at least one male gametophyte produced by suspension culture, at least one callus generated from the suspension cultured male gametophyte, at least one plantlet generated from the callus, at least one plant generated from the plantlet, and at least one seed generated from the plant.

In a third aspect, the present invention provides a culture comprising a haploid maize male gametophyte in a suspension medium, said medium containing components which allow for the cells of the male gametophyte to actively grow and divide, and to maintain their development as single cells or cell clusters.

In a fourth aspect, the present invention provides a method for the selection of maize mutants in a homozygous condition, comprising the step of in vitro screening and selection using haploid cells of a maize male gametophyte produced by suspension culture, and products produced therefrom.

In a fifth aspect, the present invention provides a method for creating genetic translocations, substitution and addition lines in maize, said method comprising the steps of (1) providing male gametophytes of interspecific and intergeneric hybrids of maize, and (2) culturing said male gametophytes in suspension, and products produced therefrom.

In a sixth aspect, the present invention provides a method for the transformation of maize, said method comprising providing haploid cells of maize male gametophyte produced by suspension culturemaize derived from a maize male gametophyte produced by suspension culture and inserting genetic material into the haploid cells by a technique selected from the group consisting of microinjection apparatus and particle bombardment, and products produced therefrom.

Donor Plant Sex Organs

The present invention may be used with any maize genotype capable of high levels of haploid and/or double haploid formation from cultured sex cells.

As used herein the term "plant" includes seed capable of being germinated into a plant; plant cells; plant protoplasts; plant cell or tissue cultures from which a plant can be regenerated; plant calli; plant clumps; and plant cells that are intact in a plant or parts of a plant, such as flowers, kernels, ears, cobs, leaves, husks, stalks, and the like.

The maize male gametophytes may be obtained from any source. Generally, the male gametophytes are obtained directly from plants which are capable of being successfully cultured *in vitro*. By "successful" is meant that *in vitro* cultured sex organs (e.g., anthers and ovules) or isolated spores produce embryo-like structures (ELS) from spores.

An exemplary technique for the generation of haploid maize plants is set forth below.

Creation of Genotype

Plants containing heterozygous genotypes may be obtained from any convenient source known to the skilled artisan. For example, the plants may be naturally heterozygous, occurring from open pollination; wild relatives of inbred lines; mutations of inbred lines; transformed inbred lines; and the progeny of crosses of inbred lines, each of which may have one or more desirable characteristics lacked by the other or which complement the other. Exemplary crosses include single crosses (i.e., two inbred lines are crossed to produce $F_1$ progeny), three-way crosses (i.e., three inbred lines are crossed: ((A X B) X C)), and double crosses (i.e., four inbred lines are crossed: (A X B) X (C X D)).

Plant breeding techniques suitable for production of such first generation hybrids are well known to those skilled in the art. Such techniques are described in Corn and Corn Improvement, Sprague (ed.), American Society of Agronomy (1977), Publication No. 18; Poelhman, Breeding Field Crops (1959); and Welsh, Fundamentals of Plant Genetics and Breeding (1981). Preferably, at least one of the parents contains commercially-important traits. Although the anther culturability phenotype may be transferred to commercially-important plants, as discussed below, it is more convenient to utilize commercially-important plants directly in the method of the present method. The presence of other traits should be selected so as not to affect the transfer of genetic factors which express an anther culture phenotype. However, as discussed infra, the anther culture trait may subsequently be transferred from highly anther culture responsive plants to other plants, such as commercially-important plants. A particularly preferred selection of plants is derived from maize plants made by a three-way cross of H99, FR16, and Pa91: (H99 X FR16) X Pa91. Seed for producing the inbred plants was obtained from Holden's Foundation Seeds, Williamsburg, IA, (H99 and Pa91) and Illinois Foundation Seed, Tolono, IL (FR16).

Anther Culture of the Donor Plant

The anthers may be removed from the plant at any suitable stage of maturity for anther culture. The stage of maturity will depend upon the particular species. Generally, the anthers will be removed when they contain microspores at the early uninucleate--late binucleate stage of development. Preferably, maize anthers are removed from the plant at between the late uninucleate-early binucleate stage of development.

Maturity of the anthers is determined microscopically by periodic sampling of plants. Microscopic techniques are well known in the art. Generally, the stage of anther development is readily determined microscopically after treatment with nuclear staining; examples of suitable stains include acetocarmine and mithramycin.

After anthers from the donor plant have been isolated, the second step involves utilizing cell culture technology to isolate and characterize cell lines which express anther culturability. The anthers may be cultured by any standard technique which provides double haploid plants. The anther culture technique employed will of course depend upon the particular species used. For a general discussion of anther culture procedures see Dunwell (1985), *supra*; Keller *et al.* (1987), *supra*; and Bajaj, In: Handbook of Plant Cell Culture , Evans *et al.* (eds.), 1:228-287 Intermating of Regenerated Plants .

After producing a population of regenerated double haploid plants as discussed above, the artisan should randomly cross the regenerated plants to produce a series of $F_1$ hybrids, and then self pollinate the $F_1$ hybrids to produce an $F_2$ population having genetic variability, i.e, segregating populations. The inventor has found, quite surprisingly, that by intermating regenerated plants of anther culture to produce an $F_1$ population and self-pollinating individuals of the $F_1$ populations to generate an $F_2$ population, the $F_2$ progeny of those plants have an anther response frequency at least 10 percent greater than the anther culture response of the donor plant.

Exemplary techniques for intermating the regenerated plants include single, three-way and double crosses of the regenerated population; the techniques for performing the crosses has hereinbefore been described and incorporated by reference. Preferably, the individuals of the regenerated population should be crossed in as many ways as possible in order to create several $F_2$ populations.

Intermating of the regenerated plants is followed by creating a segregating population to produce maximum genetic variability in the $F_2$ ($S_0$) populations. The $F_2$ population is created by self-pollinating or cross-pollinating individuals of the $F_1$

population. Any method of creating a segregating population to produce maximum genetic variability may be employed to create the $F_2$ population. Thus, the present invention contemplates pollinating individuals of the $F_1$ population by self pollination; or by being crossed with other plants such as with other members of the $F_1$ population, or even nonresponsive plants; provided that the $F_2$ progeny of those plants have an anther response frequency at least 10 percent greater than the anther culture response of the donor plant. Self-pollination of the $F_1$ individuals is preferred to maximize the release of genetic variability of the $F_1$.

An exemplary method for maintaining a segregating population is as follows: at least 50 seeds are planted and the resulting individuals are intermated (using each plant as a male and female once). See Hallauer (1987), In: Crop Species , W. R. Fehr et al. (ed.), 8:249-294. At least 25 ears from different plants should be harvested, the seed removed and mixed together. From this bulked seed mix, a random sample of seeds can be saved.

Genetic Fixation of Segregating Population

Thereafter, the segregating populations are genetically fixed. Generally, genetic fixation may be accomplished by any standard technique.

Suitable techniques include (1) providing anthers from the progeny of intermated plants and subjecting the anthers to anther culture, or (2) inbreeding the progeny of the intermated plants.

Any suitable anther culture technique which provides double haploid plants can be used. Such techniques are described above (see Dunwell (1985), supra, and Keller et al. (1987), supra).

Inbreeding involves the controlled self-pollination for several generations in order to develop true-breeding or homozygous inbred lines. Inbred lines are derived by a process of self-pollination and selection, usually over 5 or more generations ($S_1$ to $S_5$), so that allelic pairs of genes on homologous chromosome pairs are homozygous or identical.

Plants which have been self-pollinated and selected for type over many generations become homozygous at almost all gene loci and produce a uniform population of true breeding progeny. The degree of inbreeding in a line is approached at the rate of about 50 percent per generation so that by the second generation the plants are about 75 percent homozygous and by the sixth generation the plants are about 98 percent homozygous.

Thereafter, all plants derived from self-pollination, sibling pollination, or random crossing with others in the inbred line theoretically should be essentially genetically identical and, therefore, should be essentially homozygous and uniform in appearance. Consequently, a more specific embodiment of the present invention is the sibling pollination, or random crossing with others in the inbred line of a regenerated plant produced by the anther culture of a hybrid plant of the (H99 X FR16) X Pa91 cross. The inventor has found in maize a particularly important genetic factor, high anther culture (HAC) genetic factor, for enhancing the anther culturability of maize. The HAC genetic factor was created in plants resulting from the three-way cross of (H99 X FR16) X Pa91, which experiment is described below.

Preferred maize plants are highly culturable genotypes which have been deposited at the American Type Culture Collection (Bethesda, Maryland, USA): 139/39-Bulk (ATCC No. 40519), which is 139/39-01 to 139/39-14; and 139/39-DH (ATCC No. 40520).

The HAC genetic factor is such that when a series of inbred lines are intermated, the average value of a line can be used to predict the response of a given cross. This is usually a function of additive gene effects and their interaction. This is typical of quantitatively inherited traits in maize (i.e., involving the interaction of more than one gene). Individual hybrids can, however, deviate from the average performance of their parents. Thus, dominance or dominant types of epistasis may also play a role in the anther culture response.

The HAC genetic factor or equivalents thereof can increase the anther culturability of both inbred and heterotic maize plants which possess other desirable characteristics. By "equivalent" is meant to include those genetic factors which are derived from the HAC genetic factor.

A skilled artisan will appreciate that as a result of the inventor's discovery, namely the HAC genetic factor, the source of HAC genetic factor is irrelevant. Any source of DNA which provides a DNA sequence having a homologous segment to the DNA encoding the HAC genetic factor is now readily within the means of those of ordinary skill in the art. A series of probes, made from genes of all or a part of the HAC genetic factor, could be used to find homologous genes or gene segments in unknown plants, particularly maize plants. By "homologous genes or gene segments" is meant to include any DNA sequence whose mRNA hybridizes with the probes of all or a part of the HAC genetic factor, provided that the homologous segment or homologous genes produce a unique phenotype in plants, namely $F_2$ plants or their progeny, plants having an anther frequency response at least 10 percent greater than the anther culture response of the original donor plant.

By "derived" it is also intended to mean DNA sequences, as defined above, modified into altered forms. By "altered" forms is meant to include the addition, deletion, or nonconservative substitution of a limited number of various nucleotides or the conservative substitution of many nucleotides, provided that the proper reading frame is maintained.

Techniques for substitution at predetermined nucleotide sites having a known sequence are well known. Exemplary techniques include site-directed mutagenesis, the polymerase chain reaction technique, and exon shuffling. Substitution may be conducted by making nucleotide insertions, usually on the order of about 1 to about 10 nucleotides, or deletions of about 1 to about 30 nucleotides. Substitutions, deletions, insertions or any subcombination may be combined to arrive at a final construct.

However, as one aspect of the present invention is directed to intermating populations of regenerated plants, the invention is not limited to the exemplary populations deposited at the ATCC, i.e., those individuals which possess the HAC genetic factor. Methods and plants are provided for producing callus cultures, plant tissues, plants and seeds which express culturability and genetically transmit this trait to progeny.

As discussed below, a plant produced according to the present method is capable of transmitting its genetic factor for the expression of the anther culture phenotype to progeny when crossed with a plant of the same species which does not possess the improved anther culture phenotype. The progeny will display an easily defined anther culture response frequency.

Thus, the invention also relates to a plant or plants produced by, i.e. having as an ancestor, a plant containing the HAC genetic factor, as well as variants and mutants thereof. The terms "variants", "modifications", and "mutants" refer to hybrid seed or a plant produced by that hybrid which is phenotypically similar to plants containing the HAC genetic factor.

Use of the HAC genetic factor, however, is preferred because it is stable and capable of being transmitted to progeny over a number of generations. Maize plants containing the HAC genetic factor are characterized by the ability to transmit the HAC genetic factor to progeny when crossed with other maize plants which do not possess the HAC genetic factor.

The haploid plants may be converted to double haploid plants by techniques well-known in the art. Exemplary techniques are taught by C. J. Jensen (1974)In: Haploids in Higher Plants , Kasha, (ed.), pp 153-190.

Once the maize plant is selected, its tassel or ear may be harvested and pretreated as follows.

A preferred method of tassel pretreatment is set forth in Petolino and Jones (1986), *supra*. Generally, that reference teaches the following: tassels with anthers containing male gametophytes at the late uninucleate-early binucleate stage of development, as determined microscopically, may be removed from donor plants prior to emergence from the whorl. Microscopic techniques are well-known in the art. Generally, the stage of male gametophyte development is readily determined microscopically after treatment with nuclear staining; examples of suitable stains include acetocarmine and mithramycin.

Tassels are then wrapped in moist paper towels, covered with aluminum foil, and maintained at 8°C for 14 days. Before anther excision, tassels are surface sterilized for 15 minutes in a sterilizing agent, such as alcohol or a bleach, e.g., 0.5 percent sodium hypochlorite solution, followed by a sterile water rinse. Preferably, only anthers from the central portion of the main tassel branch are used.

Either the tassels or the excised anthers may be pretreated prior to removal of male gametophytes. Any particular physical or chemical pretreatment technique suitable to condition the male gametophytes for abnormal development may be employed. A preferred pretreatment method is to expose the tassel or excised anther to a temperature of between 4°C and 12°C is set forth in Petolino and Jones, (1986), *supra*.

A preferred method of ear pretreatment is set forth in Truong-Andre and Demarly (1984), Z. Pflazenzüchtg , 92:309-320. Generally, that reference teaches that the ears are harvested and most outer husks are removed after surface sterilization in a 5 to 20 percent bleach solution. After about 20 minutes, the immature ears are rinsed with sterile water. The ears may be pretreated prior to removal of the ovules by wrapping in moist paper towels, covering with aluminum foil, and maintaining the wrapped ears at 14°C for 10 days.

## IS Culture

The spores generally are then cultured in the IS culture medium. The IS culture medium contains components which allow for the spores to develop via abnormal division to proliferate into induced spores (IS). For purposes of this invention, induced spores (IS) are defined as multinuclear structures deviating from the normal developmental pattern, yet still enclosed within the exine and embryo sac.

During the first week in culture, the nuclei in many of the microspore cells degenerates leaving empty pollen grains and IS. Abnormal development of the microspores then proceeds via all four pathways reported for maize. See Miao *et al.* (1978), In: Plant tissue culture , pp 23-33. The number of IS reaches a peak during the first seven days in culture and declines thereafter.

During the first month in culture, the nuclei in many of the megaspore cells divide to form IS approximately 2 to 10 cells in size. Abnormal development of the megaspores proceeds via gynogenesis. See Yang and Zhou (1982), *supra*, and Truong-Andre and Demarly (1984), *supra*.

The IS develop further into multicellular masses (MM) and embryo-like structures (ELS). For purposes of this invention, multicellular masses (MM) are defined as structures consisting of from2 to 100 cells which have broken out of the exine and/or embryo sac. By "ELS" is meant globular masses of at least 100 cells resulting from repeated divisions of MM which are capable of continued growth and development.

By "gametophyte" is meant a microspore and/or megaspore, as well as their derivatives, e.g., IS, MM, and ELS.

Generally, length of culture will determine the stage of development of the majority of cultured spores. In the early days of the culture, spores and IS will predominantly exist. Multicellular masses generally appear at 14 to 45 days in culture, although many of the IS do not reach the MM stage. Thereafter, the MM develop into ELS, which generally first appear at 25 days in culture, although many of the MM do not reach the ELS stage.

The spores will be cultured for a period of time sufficient to have a predominant amount of MM and ELS in culture. Preferably, the sex organs will be cultured for a period of between 3 days and 35 days.

The spores are preferably cultured in the IS culture medium at a temperature ranging from 10°C to 40°C.

Generally, the spores may be cultured in a light environment ranging from complete darkness to complete light. Preferably, the spores generally will be exposed to a photoperiod ranging from 8 hours of light per 16 hours of dark to 16 hours of light per 8 hours of dark. More preferably, to prevent the beginning of substantial maturation, the spores will be cultured in complete darkness.

The light may be diffused light as well as direct light and have an intensity of between 9, micromoles per meter$^2$ per second ($\mu$mol x m$^{-2}$ x sec$^{-1}$) (500 lux) to 56 $\mu$mol x m$^{-2}$ x sec$^{-1}$ (3000 lux).

The IS culture medium components useful herein are aqueous compositions (the water constituent is deionized and distilled), containing a basal salt mixture, and at least one carbon source.

The components of the IS culture medium are preferably mixed with activated charcoal. Generally, the activated charcoal is added with the IS culture medium components before autoclaving. However, if the activated charcoal is sterilized, it may be added after the other components have been autoclaved. Generally, the activated charcoal is added in an amount sufficient to absorb any deleterious by-products. Preferably, the activated charcoal is added with the other components in an amount of between 1 gram per liter (g/l) and 10 g/l, and most preferably between 3 g/l and 7 g/l.

The activated charcoal is preferably filtered out prior to contacting the isolated spores with the IS culture medium. The activated charcoal may be filtered by any method known to the skilled artisan. An exemplary filtration technique includes passing medium through a sieve, e.g., a NalgeneTM filtration unit, or cheese cloth. Preferably, the sieve should have pores which are small enough to filter out the activated charcoal.

The basal salt mixture is a major component of the IS culture medium. Suitable basal salt mixtures include YP, N6, MS, and B5, which are taught in Plant Tissue and Cell Culture (1977),; and Chu *et al.*, (1978), Proc. Symp. Plant Tissue Cult. , pp 43-56. A preferable basal salt mixture is the YP basal salts, which should be used as taught by Ku *et al.* (1978), Proc. Symp. Plant Tissue Cult. , pp 35-42.

Exemplary carbon sources include sucrose, glucose, and fructose, with sucrose being preferred. Generally, the carbon source should be present in a concentration sufficient to support cellular growth. Preferably, the carbon source will be present in an amount of between 10 g/l and 200 g/l. Most preferably, when the carbon source is sucrose, the carbon source will be present in an amount of between 60 g/l and 90 g/l.

Generally, the IS culture medium may contain conventional organic components which are essential for cell function. Suitable organic components include conventional vitamins, amino acids, and hormones. Such organic components are taught in Street (1977), *supra*. An exemplary amino acid mixture includes casein hydrolysate. Preferably, the amino acid will be present in an amount of between 100 milligrams per liter (mg/l) and 1000 mg/l. Optionally, the IS culture medium may contain a hormone(s) in an amount of between 0.1 mg/l and 50 mg/l. Exemplary hormones include 2,4-D, dicamba, picloram, and the like. The hormones may be mixed with a solvent such as water, ethyl alcohol, hydrochloric acid, and the like.

Except for filter sterilized components, the media in the IS culture medium is normally sterilized by autoclaving, e.g., utilizing 1 kilogram per square centimeter (kg/c$^2$) (16 pounds per square inch (psi)) steam. The pH of the media prior to autoclaving normally ranges from 5.6 to 6.0.

Isolation of Gametophytes

In a preferred embodiment, the gametophytes are removed from the sex organs prior to suspension culture. Most preferably, the spores are be removed from the sex organs prior to being contacted with the IS culture medium. The purpose of removing the gametophytes from the sex organs is to remove unessential sex organ debris, e.g., anther and ovary wall.

The gametophytes may be removed from the sex organs by any suitable technique. Suitable techniques include mechanical removal or dehiscence of the gametophytes into a culture medium.

Generally, mechanical removal of male gametophytes may be accomplished by passing the anthers through a mesh screen as set forth in Lichter (1982), Z. Pflanzenphysiol. 105:427-434; or by microblending as set forth in Swanson et al., (1987), Plant Cell Reports , 6:94-97. Generally, mechanical removal of female gametophytes may be accomplished by various methods set forth in Truong-Andre and Demarly (1984), supra. This reference teaches dissecting out the individual ovules mechanically.

Generally, dehiscence comprises the shedding of gametophytes into a liquid culture medium. Anthers and ovules floating in liquid medium presumably produce conditioning factors and release them into the medium. See Sunderland et al. (1977), Nature 270:236-238.

Suspension Culture

The gametophytes are then cultured in the suspension medium. Suspension culture as defined herein consists primarily of the culturing of finely dispersed, small clusters of densely cytoplasmic cells which proliferate rapidly.

The gametophytes and, optionally, the IS culture medium may be added directly into the suspension culture. Adding the gametophytes and the IS culture medium into the suspension culture allows the less mature gametophytes (e.g., spores, and IS) to further develop into MM and ELS. Adding the IS culture with the gametophytes also provides a gradual, rather than abrupt, change in medium environment, which may be of some benefit to the development of the cells.

Initially, the volume of gametophytes added to the suspension culture determines the volume of suspension culture medium used. Generally, the volume of suspension culture medium should be sufficient to provide adequate supplies of nutrients to the gametophytes in suspension. For relatively small amounts of gametophytes (up to about $10^3$ cells), between 1 milliliter (ml) and 10 ml of suspension culture medium should be used. For relatively large amounts of gametophytes, ($10^3$ to $10^9$ cells), between 10 ml to 1000 ml of suspension culture medium should be used.

The gametophytes are incubated within a suitable range of temperatures and light intensities to cause proliferation of MM and ELS. The suspended gametophytes are cultured preferably in the dark or diffused light; and preferably at a temperature ranging from 20°C to 32°C, most preferably between 26°C to 29°C.

The suspension medium contains components which allow for the cells to actively grow and divide, and to maintain their development as single cells or cell clusters. The suspension medium components useful herein are aqueous compositions (the water constituent is deionized and distilled), containing a basal salt mixture, and at least one carbon source.

The basal salt mixture is a major component of the suspension medium. Suitable basal salt mixtures include YP, N6, MS, and B5, which are taught in Plant Tissue and Cell Culture (1977), supra; Ku et al. (1978), Proc. Symp. Plant Tissue Cult. , pp 35-42, and Chu et al. (1978), Proc. Symp. Plant Tissue Cult. , pp 43-56.

Exemplary carbon sources include sucrose, glucose, and fructose, with sucrose being preferred. Generally, the carbon source should be present in a concentration sufficient to support cellular growth. Preferably, the carbon source will be present in an amount of between 10 g/l and 200 g/l. Most preferably, when the carbon source is sucrose, the carbon source will be present in an amount of between 60 g/l and 90 g/l.

Generally, the suspension medium may contain conventional organic components which are essential for cell function. Suitable organic components include vitamins, amino acids, and hormones. Such organic components are taught in Street (1977), supra. An exemplary amino acid mixture includes case in hydrolysate. Preferably, the amino acid will be present in an amount of between 100 mg/l and 1000 mg/l.

Generally, an auxin, or hormone, may be present as a growth regulator that induces unorganized tissue development. Exemplary hormone includes 2,4-D and dicamba. The hormones may be mixed with a solvent such as water, ethyl alcohol, hydrochloric acid, and the like.

Preferably, the auxin will be present in an amount sufficient to induce cellular proliferation. Depending upon the specific hormone selected, the hormone should be present in an amount of between 0.1 mg/l and 50 mg/l. For example, relatively strong auxins, such as 2,4-D, should be present in an amount of between 0.1 parts per million (ppm) and 5 ppm. Relatively weak auxins, such as dicamba, should be present in an amount of between 1 ppm to 50 ppm. Often, hormones will be used concurrently and thus the concentrations will be adjusted accordingly.

Except for filter sterilized components, the media in the suspension medium is normally sterilized by autoclaving, e.g., utilizing 16 psi steam. The pH of the media prior to autoclaving normally ranges from 5.6 to 6.0.

The suspension of cultured gametophytes are comprised of two distinct types of cells: (a) large, vacuolated and often elongated cells, with sparse cytoplasm, and devoid of any storage starch; and (b) small, generally rounded cells, that are richly cytoplasmic and that contain a prominent nucleus and plastids with starch. Within the suspension culture, intermediate stages of the two cells types can be found.

The small, generally rounded cells undergo rapid cell divisions without any noticeable cell enlargement, producing compact and discrete groups of cells.

In culture, the single-cell gametophytes divide to form MM, and MM further develop to form ELS. The ELS, show a clear zonation in organization. Several of the peripheral layers are composed of small, cytoplasmic cells with conspicuous storage starch, and a central mass of cells that are slightly larger in size, less densely cytoplasmic, and contain very little starch.

When an ELS is separated it yields single cells and/or MM. Individual cells and cells in small MM stay viable in culture longer than cells in large MM or ELS, because the cells in the middle of the large MM or ELS tend to become starved for oxygen and nutrients. Consequently, it is beneficial to separate the large MM or ELS into single cells or small MM. In the suspension cultures, the embryogenic clusters undergo constant cleavage into single cells or small groups of MM which continue to divide.

Any suitable means of separating, but not damaging, the cells in the larger MM and ELS may be used. For example, the flasks may be placed on an agitation means and agitated at a rate sufficient to adequately bath the gametophytes in the suspension culture and to maintain the gametophytes as single cells or smaller MM. Generally, the flasks are agitated at a rate of between 50 revolutions per minute (rpm) and 175 rpm. Exemplary agitation means include orbital shakers, horizontal shakers, and gentle tumbling tubes.

Aging of the suspensions during the early stages, and the formation of a viscous, mucilaginous substance in the medium, are helpful to condition the media to promote beneficial embryogenic growth. Generally, no manipulation of the culture is immediately necessary.

The maintenance of suspensions is determined by the rate of cell division of the gametophytes.

Initially, no subculturing is necessary. It is suggested, however, that subculturing be done just prior to when the culture medium begins to turn dark or black. Removal of supernatant medium containing phenolic substances, enlarged, vacuolated cells, and other cellular debris is critical. Generally, the suspension, when predominantly comprised of spores and IS, should be subcultured from between 7 days and 21 days. Thereafter, the suspension, when predominantly, comprised of MM and ELS, should be subcultured from between 3 days and 14 days.

Generally, each subculture should be replenished with fresh suspension medium in a volume sufficient to give adequate nutrients to the cells. Preferably, each subculture of suspension medium should be replenished with fresh to restore the volume of the subculture to the volume of the suspension from which it was cultured.

Generally, a volume of the supernatant of the suspension culture should be removed. The remaining volume of suspension culture, and in fact the removed supernatant, are preferably mixed with fresh suspension medium to create two suspensions from the original suspension. Thereafter, each suspension may likewise be further subcultured into two suspensions.

Dilution ratios used during subculture are very important. Each suspension may be maintained by subculture at between a 1:1 and a 1:7 dilution (suspension inoculum:fresh medium), preferably between a 1:2 and a 1:4 dilution.

For a general discussion of subculturing techniques, refer to Vasil and Vasil (1981), Ann. Bot. , 47:669-678.

Callus Induction

Random samples of the suspended gametophytes may be then selectively cultured on the callus induction medium to form callus, or may be added directly to a plant regeneration medium.

By "callus" is meant cells proliferating in a more or less disorganized manner without differentiating into organized structures (roots, leaves, etc.).

The gametophytes should be cultured on the callus induction medium within a suitable range of temperatures and light intensities to foster the formation of callus. The gametophytes are cultured in the callus induction medium preferably in the dark; and preferably at a temperature ranging from 20° C to 32° C, most preferably from 26° C to 29° C.

The callus induction medium components useful herein are aqueous compositions (the water constituent is deionized and distilled), containing a basal salt mixture, at least one carbon source, and at least one plant growth hormone.

The basal salt mixture is a major component of the callus induction medium. Exemplary basal salt mixtures include the YP, N6, MS, and B5 basal salts, which are taught in Street (1977), *supra*; and Chu *et al.*, (1978), *supra*.

Exemplary carbon sources include sucrose, glucose, and fructose, with sucrose being preferred because it is so widely utilizable by most tissue cultures. Generally, a carbon source should be present in a concentration sufficient to support cellular growth. Preferably, the carbon source will be present in an amount of between 10 g/l and 200 g/l. Most preferably, when the carbon source is sucrose, the carbon source will be present in an amount of between 10 g/l and 40 g/l.

Generally, the plant growth hormone will be present in a concentration sufficient to induce callus proliferation as taught in Street (1977), *supra*. Preferably, the hormone will be present in an amount of between 0.1 mg/l and 10 mg/l. Exemplary hormones include dicamba, 2,4-D, and picloram. The hormones may be mixed with a solvent such as water, ethyl alcohol, hydrochloric acid, and the like.

Generally, the callus induction medium may contain other conventional organic components, such as vitamins and amino acids, as taught in Street, 1977. An exemplary source of vitamins is the N6 formulations of vitamins (see Ku (1978), *supra*). Amino acid components preferably include enzymatic casein hydrolysate and L-proline.

The callus induction medium may contain a gelling agent. Suitable gelling agents include, for example, agar at a level of between 0.6 percent and 0.8 percent; Gelrite at a level of between 0.1 percent and 0.2 percent; and agarose (pure basis) at a level of between 0.2 percent and to 0.4 percent.

Except for filter sterilized components, the media in the callus induction medium is normally sterilized by autoclaving, e.g., utilizing 1 kg/cm$^2$ (16 psi) steam. The pH of the media prior to autoclaving normally ranges from 5.6 to 6.0.

Generally, the samples of gametophytes in suspension will be cultured in the callus induction medium until displaying a compact, nodular appearance, or going through organogenesis, so-called "conversion effect", to form converted tissue. Preferably, the samples of gametophytes in suspension are cultured in the callus induction medium for a period of at least 7 days, and most preferably between 14 and 60 days.

Once established, the compact nodular callus or converted tissue is preferably contacted with a chromosome doubling agent in an amount sufficient to induce chromosome doubling. By "chromosome doubling agent" is meant a mitotic poison, i.e., a chemical capable of interfering with mitosis. For a detailed discussion of chromosome doubling agents see Jensen (1974), *supra*. Exemplary chromosome doubling agents include nitrous oxide and colchicine.

Plant Regeneration

Either selected callus from the callus induction medium, or samples of gametophtyes in suspension, may be transferred to a semi-solid or liquid regeneration medium.

As used herein the term "plant" includes seed capable of being germinated into a plant; plant cells; plant protoplasts; plant cell or tissue cultures from which a plant can be regenerated; plant calli; plant clumps; and plant cells that are intact in a plant or parts of a plant, such as flowers, kernels, ears, cobs, leaves, husks, stalks, and the like.

Incubation of the callus or samples of gametophtyes in suspension is carried out within a suitable range of temperatures and light intensities to foster the formation of plantlets. The callus or samples of gametophtyes in suspension are preferably cultured in the plant regeneration medium at a temperature ranging from 26°C to 29°C. The callus or samples of gametophtyes in suspension preferably will be exposed to a photoperiod ranging from 8 hours of light per 16 hours of dark to 18 hours of light per 6 hours of dark; more preferably, from 16 hours of light per 8 hours of dark. The light may be diffused light as well as direct light and have an intensity of between 50 $\mu$mol x m$^{-2}$ x sec$^{-1}$ (250 foot candles) to 100 $\mu$mol x m$^{-2}$ x sec$^{-1}$ (500 foot candles).

The plant regeneration medium may contain components which foster the growth of plantlets from the callus or samples of gametophtyes in suspension . The plant regeneration medium components useful herein are aqueous compositions (the water constituent is deionized and distilled), containing a basal salt mixture and at least one carbon source.

The basal salt mixture is a major component of the plant regeneration medium. Exemplary basal salt mixtures include SH (described in Shenk (1972), Can. J. Bot. , **50**:199); MS (described in Murashige *et al.* (1962), Physiol. Plant , **15**:473); B5 (described in Gamborg (1968), Exp. Cell Res. , **50**:148); BL (described in Blaydes (1966), Physiol. Plant , **19**:748-753); WH (described in White (1963), The Cultivation of Animal and Plant Cells ); and N6 (described in Chih-Ching *et al.*, (1975), Scientia Sinica , 9:659-668). Preferably, the SH basal salts are employed.

Exemplary carbon sources include sucrose, glucose, and fructose, with sucrose being preferred Generally, a carbon source should be present in a concentration sufficient to support cellular growth.Preferably, the carbon source will be present in an amount of between 10 g/l and 200 g/l. Most preferably, when the carbon source is sucrose, the carbon source will be present in an amount of between 5 g/l and 50 g/l.

The plant regeneration medium may beneficially contain a gelling agent. Suitable gelling agents include, for example, agar at a level of between 0.6 percent and 0.8 percent; Gelrite at a level of between 0.1 percent and 0.2 percent; and agarose (pure basis) at a level of between 0.2 percent and to 0.4 percent.

Except for filter sterilized components, the media in the ·plant regeneration medium is normally sterilized by autoclaving, e.g., utilizing 1 kg/cm² (16 psi) steam. The pH of the media prior to autoclaving normally ranges from 5.6 to 6.0.

The callus or samples of gametophtyes in suspension are incubated in the plant regeneration medium until producing root or shoot structures, e.g., between 2 to 5 nodes. Generally, incubation is conducted for a period of between 1 to 4 weeks.

It is preferable to subculture individual plantlets to fresh medium of the same composition.

From the plant regeneration medium, the plantlets are planted in soil or potting medium, e.g., in a greenhouse or in the field and under ambient normal growth conditions the plantlets develop into whole fertile plants which flower and develop seeds.

Finally, the seeds are recovered to provide further generations of plants from which seeds can be recovered. The seeds are recovered from the plants propagated above or from descendants thereof by harvesting the seed pods and separating the seeds therefrom, for example, by hand or by using a thresher or combine.

The post plant-regeneration steps are readily carried out by conventional plant husbandry techniques.

## Uses

Because the gametophytes are haploid, the suspension culture has several advantages over convention anchorage-dependent cultures. These advantages include the production of a population of single haploid cells capable of plant regeneration through embryogenesis and the avoidance of potentially undesirable interactions between developing male gametophytes.

Because the gametophytes are haploid, suspension cultures provide unique possibilities for genetic manipulation. More specifically, the use of suspension culture of haploids has many potential applications in cellular biology research, i.e., rapidly producing large numbers of inbred lines for commercial evaluation.

For example, one application is the efficient and quick evaluation of the outcome and usefulness of a cross if a large number of progeny became available within a short time after the cross was made. Such cultures provide a clonal population of plants derived from the embryo, and can be used for progency testing and selection within a matter of months.

Another application is the production of desirable genetic translocations, substitution and addition lines through the culture of gametophytes of interspecific and intergeneric hybrids. The production of haploids and thereafter homozygous lines is a significant ·methodology for selecting rare genotypes, including those with recessive characters, and to include them in new combinations of crosses. Suspension culture permits the *in vitro* screening and selection using haploid cells, and to obtain mutants in a homozygous condition.

A third application is the transformation of maize. The transformation of maize has been restricted by the limitations of presently available gene-transfer systems.

One method for transformation of maize is the direct intake of DNA into protoplasts. For a discussion of techniques fro producing protoplasts Rhodes *et al., supra*.

Another method for transformation of maize is for the insertion of DNA directly into the gametophytes. Suspension culture may be used to provide a ready source of embryonic cells for insertion of DNA into the gametophytes via, for example, the gene gun technology. See Klein *et al.*, Biotech , **6**:559-563.

Exemplary techniques for the delivery of rDNA (recombinant DNA) providing increased androgenesis include microinjection (see Crossway et al. Mol. Gen. Genet. , 202: 179-185), or the gene gun technology (see Klein et al., Biotech , 6:559-563).

Conventional technologies for introducing biological material into living cells include particle bombardment technology, microinjection mechanisms, infectious agents, and techniques for protoplast transformation (e.g., electroporation, uptake mechanisms, and fusion). These techniques provide, upon chromosome doubling, a plant homozygous for the rDNA.

Relatively recently, particle bombardment technology has been developed to deliver substances into cells of intact tissues via particle bombardment.

One particle bombardment technology is set forth in *High-velocity Microprojectiles for De-*

*livering Nucleic Acids into Living Cells* (1987), T. M. Klein, E. D. Wolf, R. Wu and J. C. Sanford, Nature , 337; and *Delivery of Substances into Cells and Tissues using a Particle Bombardment Process*, J. C. Sanford, T. M. Klein, E. D. Wolf, and N. Allen, Particular Sci. and Technol. , 5:27-37. These references teach the acceleration of at small high-density, tungsten particles (microprojectiles) may be accelerated to high velocity via the following embodiments: (1) a macroprojectile (plastic bullet) and stopping plate, (2) a transferred mechanical pulse, (3) a gas (e.g., air) discharge, and (4) a centripetal acceleration system.

Another particle bombardment technology is set forth in published EP 0 270 356. Generally, the reference teaches an apparatus for injecting carrier particles carrying DNA into living cells characterized in that it comprises: a spark discharge chamber; two electrodes extending into the spark discharge chamber and spaced apart by a spark gap, the electrodes being adapted for attachment to an external source of high voltage discharge; a carrier sheet held spaced above the spark discharge chamber, the carrier sheet receiving the carrier particles thereon; a retaining screen fixed in place above the carrier sheet; and a target surface held spaced above the retaining screen and carrying the cells so that a spark discharge generating a shock wave in the discharge chamber will accelerate the carrier sheet into the retaining screen so that the carrier particles are accelerated into the cells on the target surface.

Another technique is a direct method for the transfer of chromosomes by microinjection. For a general discussion of microinjection techniques, see *Methods for Microinjection of Human Somatic Cells in Culture*, (1973), E.G. Diacumakos, In: Methods in Cell Biology , D.M. Prescott (ed.), Academic Press, NY, pp. 287-311; *Microinjection of Tissue Culture Cells* (1973), M. Graessman and A. Graessman, Methods in Enzymology , 101:482-492; Crossway A., Oakes J. V., Irvine J. M., Ward B., Knauf V. C., Shewmaker C. K. (1986), *Integration of Foreign DNA following Microinjection of Tobacco Mesophyll Protoplasts*, Mol. Gen. Genet. , 202:179-185; Crossway A., Hauptli H., Houck C. M., Irvine J. M., Oakes J. V., Perani L. A. (1986), *Micromanipulation Techniques in Plant Biotechnology*, Biotechniques , 4:320-334; Reich T. J., Iyer V. N., Scobie B., Miki B. L. (1986), *A Detailed Procedure for the Intranuclear Microinjection of Plant Protoplasts*, Can. J. Bot .; and Reich T. J., Iyer V. N., Miki B. L. (1986), *Efficient Transformation of Alfalfa Protoplasts by the Intranuclear Microinjection of Ti Plasmids*, Bio/Technology , 4:1001-1004.

In addition to the systems mentioned above, there exist several infectious agents which can deliver nucleic acids into cells. Of primary importance are the *Agrobacterium* vectors for dicot plant cells (*Genetic Transformation in Higher Plants* (1986), R. T. Fraley, S. G. Rogers, and R. B. Horsch, CRC Crit. Rev. Plant Sci. , 4: 1-46; and the retroviral vectors for animal cells (*Prospects for Gene Therapy* (1984), F. W. Anderson, Science , 226:401-409).

Retroviruses (RNA viruses) can be used to deliver genes into animal cells. When the virus enters the cell its RNA acts as a template for reverse transcription of complementary DNA which will integrate into the genome of the host cell. This DNA can be isolated and inserted into a plasmid. This plasmid, with additional genes added, can be used to transform cells with the aid of helper retroviruses.

Electroporation is a method for introducing a variety of molecules into cells and protoplast isolated therefrom by subjecting them to brief high-voltage electric pulses. For a general discussion of electroporation, see *Electroporation of Eukaryotes and Prokaryotes: A General Approach to the Introduction of Macromolecules into Cells*, (1988), K. Shigekawa and W. J. Dower, Biotechniques , 6 :742; *High-voltage Electroporation of Bacteria: Genetic Transformation of Campylobacter jejuni with Plasmid DNA*, (1988), J. C. Miller, W. J. Dower, L. S. Tomkins, Proc. Natl. Acad. Sci. USA , 85 :856-860; and *A Simple and Rapid Method for Genetic Transformation of Lactic Streptococci by Electroporation*, (1988), I. G. Powell, M. G. Achen, A. J. Hillier, B. E. Davidson, Appl. Environ. Microbiol. , 54 :655-660.

One technique for uptake is the enhancement of membrane permeability by use of calcium (Ca) (*Calcium Dependent Bacteriophage DNA Infection* (1972), M. Mandel and A. Higa, J. Mol. Biol. , 53:159-162); and temperature shock (*Frozen-thawed Bacteria as Recipients of Isolated Coliphage DNA* (1972), S. Y. Dityatkin, K. V. Lisovskaya, N. N. Panzhava, B. N. Liashenk, Biochimica et Biophysica Acta , 281:319-323).

A second technique for uptake is the use of surface-binding agents such as polyethylene glycol (PEG). For a general discussion of surface-binding agent, see *High Frequency Transformation of Bacillus subtilis Protoplasts by Plasmid DNA* - (1972), S. Chang, and S. N. Cohen, Mol. Gen. Genet. , 168: 111-115; *In vitro Transformation of Plant Protoplasts With Ti-plasmid DNA* (1982), F. A. Krens, L. Molendijk, G. J. Wullems, and R. A. Schilperoort, Nature , 296:72); or such as calcium phosphate (*A New Technique for the Assay of Infectivity of Human Adenovirus 5 DNA* (1973), F. L. Graham, and A. J. Van der Eb, Virology , 52: 456; *Transformation of Mammalian Cells with Genes from Procaryotes and Eucaryotes* (1979), M. Wigler, R. Sweet, G. K. Sim, B. Wold, A. Pellicer, E. Lacy, T. Maniatis, S. Silverstein, and R. Axel, Cell , 16:777.

A third technique for uptake is the phagocytosis of particles into a protoplast. Suitable particles include liposomes (Liposome-mediated Transfer of Plasmid DNA into Plant Protoplasts - (1982), H. Uchimiya, T. Ohgawara, and H. Harada, In: A. Fujiwara (ed.), Proc. 5th Intl. Cong. Plant Tissue and Cell Culture , Jap. Assoc. for Plant Tissue Culture, Tokyo, pp. 507-508); organelles (Transplantation of Chloroplasts into Protoplasts of Petunia (1973), I. Potrykus, Z . Pflanzenphysiol. , 70:364 -366); or bacteria (Plant Cell Protoplasts Isolation and Development (1972), E.C. Cocking, Ann. Rev. Plant Physiol. , 23:29-50).

Fusion can be induced with electric currents, PEG, and Sendai virus particles. For a general discussion of cell fusion, see Methods Using HVJ (Sendai Virus) for Introducing Substances into Mammalian Cells (1980), T. Uchidaz, M. Yamaizumi, E. Mekada, Y. Okada, In: Introduction of Macromolecules Into Viable Mammalian Cells , C. Baserga, G. Crose, and G. Rovera (eds.) Wistar Symposium Series, Vol. 1, A. R. Liss Inc., NY, pp. 169-185; and H. Harris, Cell Fusion: The Dunham Lectures (1970), Oxford University Press .

The following examples are presented to further illustrate but not limit the scope of this invention. All parts and percentages are by weight unless otherwise indicated.


Example


The maize genotype used for donor plants was (139/39-02), a highly androgenic $S_2$ line developed by intermating anther-derived doubled haploids (see Petolino et al., (1988), Theor. Appl. Genet. , 76: 157 -159). Tassels from the donor plants were harvested and pretreated, following previously published procedures (see Petolino and Jones (1986), supra).

Anthers from the main tassel branch were inoculated (30 anthers per 60 x 20 mm plastic Petri dish) into 10 ml of anther culture medium. Four dishes were obtained from each tassel harvested.

The IS culture medium consisted of YP basal salts (see Ku (1978), supra) with the addition of 60 g/l sucrose, 5 g/l activated charcoal 500 mg/l casein hydrolysate, and 0.1 mg/l 2,3,5-triiodobenzoic acid adjusted to pH 5.8. Prior to use, the medium was filtered to remove the activated charcoal. Specifically, the activated charcoal was removed by filtration with a commercially available filter sterilization unit having a pore size of 5 microns.

All nutrient media were sterilized by autoclaving and all culture procedures were carried out under aseptic conditions. The pH of the media prior to autoclaving ranged from 5.6 to 6.0.

The spores were preferably cultured in the IS culture medium at a temperature of about 28 ° C. The spores were maintained in the dark.

After 10 days, the IS culture was passed through a mesh screen, and the anther wall and other cellular debris discarded from the anther culture medium, which contained isolated spores, IM, and MM, and ELS.

The contents of each of the four petri dishes (including the gametophytes and IS culture medium) were emptied into a 125 ml Erlenmeyer flask containing 10 ml of suspension medium.

The suspension medium contained MS basal salts, MS vitamins, FE-EDTA, 30 g/l sucrose, and 2 mg/l 2,4-D (See Cell Culture and Somatic Cell Genetics of Plants , Indra K. Vasil (ed.), 1:152-158.

All nutrient media were sterilized by autoclaving and all culture procedures are carried out under aseptic conditions. The pH of the media prior to autoclaving normally ranges from 5.6 to 6.0.

The flasks contained the suspension cultures awere maintained on a orbital gyratory shaker (model G10 gyratory shaker, commercially available from the New Brunswick Scientific Co, Inc., Edison, NJ). The shaker was set at 150 rpm and the cells were incubated in the dark at 28° C.

The growth, color and texture of the suspension cultures was dependent upon the ratio of the two component cell types. It contained a large population of enlarged and elongated, thick-walled cells which were devoid of starch. In addition, it contained a few groups of small, round, richly cytoplasmic, and starch-containing cells.

For the first seven (7) days, no manipulation of the culture was necessary. After 1 week, the flask was removed from the shaker, the cells were allowed to settle for 1 minute. Subculturing was performed by slowly draining 15 ml of the supernatant medium from the flask. The draining of supernatant medium removeed most of the large, vacuolated, and nondividing cells which were sloughed off into the medium along with other cellular debris.

The drained supernatant was then placed into a second flask with 30 mls. of fresh suspension culture medium. Fifteen ml of fresh medium was added to the first flask to replace the drained supernatant. Both the first and second flasks were then subcultured.

After 60 days, the cultures appeared dull-white in color, and became very thick, mucilaginous and jelly-like, and it becames increasingly difficult to remove the medium by draining. It contained a large population of enlarged and elongated, thick-walled cells which are devoid of starch. In addition, it contained a few groups of small, round, richly cytoplasmic, and starch-containing cells.

These richly cytoplasmic cells were termed embryogenic cells and with further subculture their number increased. Routine subculturing and maintanence were throughout the life of the subculture.

At various times after being cultured, unwashed suspension cultures contained gametophytes from each subculture were plated on callus induction medium for the development of calluses. Such suspensions were not washed by draining or even centrifugation owing to the presence of a thick mucilaginous substance. The suspension cultures were noted 3-4 days after subculture, because a majority of the cells were actively dividing in a so-called "log" or "exponential growth" phase.

The callus induction medium consisted of N6 major salts and vitamins (see Chu (1978), *supra*), B5 minor salts (see Gamborg *et al.* (1968), *supra*), 30 g/l sucrose, 2.9 g/l L-proline, 100 mg/l myo-inositol, 100 mg/l casein hydrolysate, 2.5 mg/l dicamba, 0.1 mg/l 2,4 -D, and 8 g/l TC-agar (obtained from Hazleton Research Products, Inc., Lenexa, Kansas, USA).

All nutrient media were sterilized by autoclaving and all culture procedures were carried out under aseptic conditions. The pH of the media prior to autoclaving normally rangeed from 5.6 to 6.0.

The gametophytes were preferably cultured in the callus induction medium at a temperature of about 28 °C. The gametophytes were maintained in the dark.

After 2 to 3 weeks, callus displaying a compact, nodular appearance, or converted tissue from each callus induction medium were each transferred to a plant regeneration medium and maintained in a 16 hours of light per 8 hours of dark photoperiod from cool-white light fluorescent lamps (60 $\mu$mol m$^{-2}$ sec$^{-1}$) (300 footcandles). The regeneration medium consisted of SH basal salts (Schenk and Hildebrandt (1972), *supra*) and 10 g/l sucrose.

All nutrient media were sterilized by autoclaving and all culture procedures were carried out under aseptic conditions. The pH of the media prior to autoclaving normally rangeed from 5.6 to 6.0.

· The callus were cultured in the plant regeneration medium at a temperature of about 28°C. The callus were exposed to a photoperiod of 16 hours of light per 8 hours of dark. The light was diffused as well as direct and had an intensity of 500 lux to 3000 lux.

After 4 weeks, individual plantlets were transferred from each plant regeneration medium to 10 x 25 mm culture tubes containing fresh regeneration medium for further development before transplanting to soil. After root formation, the plantlets were transferred to soil and grown to maturity in the greenhouse at ambient temperature. The plantlets developed into whole fertile plants which flower and set seed and the seeds matured into viable seeds.

Various modifications of the invention in addition to those shown and described herein will become apparent to those skilled in the art from the foregoing description. Such modifications are intended to fall within the scope of the appended claims.

## Claims

1. A method for the culture of maize male gametophytes comprising the step of:

(a) providing at least one haploid maize male gametophyte from at least one haploid donor maize plant which is capable of being successfully *in vitro* cultured, wherein the donor maize plant is produced by (i) providing anthers from at least one heterozygous donor maize plant, (ii) regenerating, from the anthers obtained from the donor plant, at least two microspore-derived plants capable of being intermated, (iii) intermating the regenerated plants to produce an F$_1$ population, and (iv) self-pollinating or cross-pollinating individuals of the F$_1$ population to generate at least one F$_2$ population;

(b) culturing a haploid maize male gametophyte in a suspension medium, said medium containing components which allow for the cells of the male gametophyte to actively grow and divide, and to maintain their development as single cells or cell clusters.

2. The method of Claim 1, wherein the haploid maize male gametophyte is an isolated male gametophyte obtained by the following steps:

(a) providing at least one anther containing at least one microspore from at least one haploid donor maize plant which is capable of being successfully *in vitro* cultured;

(b) culturing the microspore in an IS medium for a time period sufficient to induce abnormal microspore division of at least one microspore to form a male gametophyte; and

(c) isolating at least one male gametophyte from the IS medium.

3. The method of Claim 3, wherein the haploid donor plant is selected from the group consisting of 139/39-DH and 139/39-Bulk.

4. The method of Claim 1 comprising the further step of incubating the male gametophyte on a plant regeneration medium to foster the germination of plantlets.

5. The method of Claim 1 comprising the further step of incubating the male gametophyte in a callus induction medium to foster development of callus capable of being regenerated into at least one maize plant.

6. The method of Claim 5 comprising the further step of incubating the callus on a plant regeneration medium to foster the germination of plantlets.

7. The method of any one of Claims 4 or 6 comprising the step of cultivating at least one plantlets into at least one whole fertile plant.

8. The method of Claim 7 comprising the further step of cultivating at least one whole fertile plant or a descendant thereof whereby it bears seeds.

9. At least one male gametophyte cultured by the method of any one of Claims 1 through 8.

10. At least one protoplast derived from a the male gametophyte of Claim 9.

11. A callus produced by the method of Claim 5.

12. A plantlet produced by the method of any one of Claims 4 or 6.

13. A plant produced by the method of Claim 7.

14. A seed produced by the methods of any one of Claims 8 or 12.

15. The maize seed of Claim 14, wherein the seed is a double haploid.

16. A culture comprising a haploid maize gametophyte in a suspension medium, said medium containing components which allow for the cells of the male gametophyte to actively grow and divide, and to maintain their development as single cells or cell clusters.

17. A suspension culture of Claim 16, wherein the haploid male maize gametophyte is selected from the group of microspore, IS, MM and ELS.

18. A method for the selection of maize mutants in a homozygous condition, comprising the step of *in vitro* screening and selection using haploid cells of maize derived from the male gametophyte of Claim 9 or cells derived therefrom.

19. A method for creating genetic translocations, substitution and addition lines in maize, said method comprising the steps of (1) providing male maize gametophytes of Claim 9 or cells derived therefrom, and (2) culturing said gametophytes in suspension.

20. A method for the transformation of maize, said method comprising providing the male maize gametophyte of Claim 9 or cells derived therefrom, and inserting genetic material into the haploid cells.

21. A product derived from any one of the methods of Claims 18-20.